# EUROPEAN PATENT SPECIFICATION

(11) **EP 2 777 692 B1**
(45) Date of publication and mention of the grant of the patent: **02.05.2018**
(21) Application number: 14158640.4
(22) Date of filing: 10.03.2014
(51) Int. Cl.: A61K 9/00

(54) **Composition for enhancing transdermal absorption of drug and patch preparation**
Zusammensetzung zur Verstärkung der transdermalen Absorption einer Arzneimittel- und Pflasterherstellung
Composition pour améliorer l'absorption transdermique d'un médicament et d'une préparation de timbre

(30) Priority: 11.03.2013 JP 2013048450
(43) Date of publication of application: 17.09.2014
(73) Proprietor: Nitto Denko Corporation, Ibaraki-shi, Osaka 567-8680 (JP)
(72) Inventor: Mukobata, Tsuyoshi, Ibaraki-shi, Osaka 567-8680 (JP); Okazaki, Arimichi, Ibaraki-shi, Osaka 567-8680 (JP); Hanatani, Akinori, Ibaraki-shi, Osaka 567-8680 (JP); Sakamoto, Sachiko, Ibaraki-shi, Osaka 567-8680 (JP)
(74) Representative: J A Kemp

(56) References cited:
- EP-A2- 0 368 409
- US-A1- 2009 041 680
- US-B1- 6 635 674

## Description

### TECHNICAL FIELD OF THE INVENTION

The present invention relates to a composition for enhancing transdermal absorption of a basic drug, which shows a superior transdermal drug absorption-enhancing effect.

### BACKGROUND OF THE INVENTION

As a drug administration method aiming at a systemic action, oral administration and injection are generally used widely. In oral administration, liver metabolism (first pass effect) is inevitable and, in administration by injection, blood drug concentration cannot be maintained easily, since drugs having a short biological half-life require frequent administration. On the other hand, transdermal absorption preparation is advantageous in that it overcomes the above-mentioned defects, can maintain stable blood drug concentrations, can reduce administration frequency, can improve compliance, permits easy interruption of administration and the like.

However, since many drugs show low transdermal absorbability and there are not many drugs actually formulated into transdermal absorption preparations, a means for increasing the transdermal absorbabilty of a drug is needed. Therefore, it has generally been tried to add a transdermal absorption promoter to a base material. For example, WO2000/53226 discloses that a composition containing a fatty acid having 8-15 carbon atoms or higher alcohol having 8-12 carbon atoms and polyvalent alcohol improves transdermal absorbability of drugs as a transdermal absorption promoting composition. Moreover, JP-A-5-946 discloses that a composition containing a fatty acid or aliphatic alcohol having 8-12 carbon atoms and polyvalent alcohol improves transdermal absorbability of drugs.

However, according to the study of the present inventors, such compositions that allegedly improve transdermal absorbability of conventional drugs failed to provide stable transdermal absorbability since volatilization of solvent changes the composition, or showed a transdermal absorption-enhancing effect only for a particular drug, or application to nonaqueous adhesive patch was difficult, and only a few of them showed a sufficiently superior absorption-enhancing effect and broad utility.

US 6635674 relates to an anti-inflammatory and analgesic pharmaceutical preparation for external use which comprises a non-steriodal anti-inflammatory drug. EP 0368409 describes compositions for the transdermal delivery of buprenorphine salts. US 2009/0041680 describes foamable pharmaceutical and cosmetic compositions with potentially enhanced skin delivery.

### SUMMARY OF THE INVENTION

The present invention has been made in view of the above-mentioned situation and aims to provide a composition for enhancing transdermal absorption of a basic drug, which shows a superior transdermal drug absorption-enhancing effect, and a patch preparation showing superior transdermal absorbability of a basic drug.

The present inventors have conducted intensive studies in an attempt to solve the aforementioned problems and found a combination of polyvalent alcohol having 3 - 8 carbon atoms, an organic acid having 12 - 20 carbon atoms and higher alcohol having 12 - 20 carbon atoms in the amounts defined below extremely advantageously acts on the improvement of transdermal absorbability of a drug. They have conducted further studies based on the above findings, and completed the present invention.

Accordingly, the present invention provides the following.
[1] A composition for enhancing transdermal absorption of a basic drug, comprising a polyvalent alcohol having 3 to 8 carbon atoms, an organic acid having 12 to 20 carbon atoms, and a higher alcohol having 12 to 20 carbon atoms, wherein the amount of the polyvalent alcohol is 80-98 parts by weight per 100 parts by weight of the total weight of the polyvalent alcohol, higher alcohol and organic acid, and the weight ratio of the higher alcohol and organic acid (higher alcohol:organic acid) in the remaining part is 0.01:99.99-99.99:0.01.
[2] The composition of the above-mentioned [1], wherein the higher alcohol having 12 to 20 carbon atoms is one or more kinds selected from the group consisting of lauryl alcohol, myristyl alcohol, hexyldecanol, oleyl alcohol and octyldodecanol.
[3] The composition of the above-mentioned [1], wherein the higher alcohol having 12 to 20 carbon atoms is a higher alcohol having 14 to 20 carbon atoms.
[4] The composition of any one of the above-mentioned [1] to [3], which is for a patch preparation.
[5] A patch preparation comprising a support and a drug-containing adhesive layer or drug reservoir layer on one surface of the support, wherein the layer comprises the composition of any one of the above-mentioned [1] to [3] and a basic drug.
[6] Use of a composition according to any one of the above-mentioned [1] to [4] to enhance transdermal absorption of a basic drug.

The composition for enhancing transdermal absorption of a basic drug of the present invention shows a superior transdermal absorption-enhancing effect for a basic drug. Using such composition, a matrix type or reservoir type patch preparation showing superior transdermal absorbability of a basic drug can be realized, and the effect thereof becomes remarkable.

### BRIEF DESCRIPTION OF THE DRAWINGS

Fig. 1 is a schematic sectional view of one embodiment of the matrix type patch preparation of the present invention.
Fig. 2 is a schematic sectional view of one embodiment of the reservoir type patch preparation of the present invention.
In the Figures, 1 is a release liner, 2 is a drug-containing adhesive layer, 2' is an adhesive layer, 3 is a drug permeation control film, 4 is a drug reservoir layer, and 5 is a support.

### DETAILED DESCRIPTION OF THE INVENTION

The present invention is explained in the following by referring to the preferable embodiments thereof.

The composition for enhancing transdermal absorption of a basic drug of the present invention (hereinafter to be also simply referred to as "the composition of the present invention") mainly comprises a polyvalent alcohol having 3 - 8 carbon atoms, an organic acid having 12 - 20 carbon atoms and a higher alcohol having 12 - 20 carbon atoms, in the amounts described above.

As the polyvalent alcohol having 3 to 8 carbon atoms, a divalent or trivalent alcohol can be used, and the structure thereof is not particularly limited. Specific examples include propylene glycol, butylene glycol, glycerol, dipropylene glycol, octanediol and the like. Of these, one having 3 or 4 carbon atoms is preferable, and particularly preferred are propylene glycol and butylene glycol. One or more kinds of such polyvalent alcohol having 3 to 8 carbon atoms can be used.

As for the higher alcohol having 12 to 20 carbon atoms, the structure thereof is not particularly limited, and any higher alcohol can be used. For example, any of the linear and branched chain higher alcohols may be used, which may have one or more unsaturated bonds. Specific examples thereof include lauryl alcohol, myristyl alcohol, cetyl alcohol, hexyldecanol, stearyl alcohol, isostearyl alcohol, oleyl alcohol, arachyl alcohol, octyldodecanol and the like. Among these, lauryl alcohol, myristyl alcohol, hexyldecanol, oleyl alcohol and octyldodecanol are preferable, and lauryl alcohol and oleyl alcohol are particularly preferable. A higher alcohol having 17 to 19 carbon atoms, for example, oleyl alcohol, is particularly preferable since a transdermal absorption-enhancing effect can be obtained irrespective of the kind of organic acid. One or more kinds of such higher alcohol having 12 to 20 carbon atoms can be used.

As the organic acid having 12 to 20 carbon atoms, a monovalent, divalent or trivalent organic acid can be used, and the structure thereof is not particularly limited, and any organic acid can be used. Specific examples thereof include lauric acid, myristic acid, palmitic acid, stearic acid, isostearic acid, oleic acid, linoleic acid, arachidic acid and the like, and myristic acid, isostearic acid and oleic acid are preferable. One or more kinds of such organic acid having 12 to 20 carbon atoms can be used.

An organic acid having 12 to 20 carbon atoms (particularly organic acid having 14 to 20 carbon atoms) is preferably used in combination with a higher alcohol having 14 to 20 carbon atoms (e.g., myristyl alcohol, hexyldecanol, oleyl alcohol, octyldodecanol etc.), since the transdermal absorption-enhancing effect becomes superior. Here, the higher alcohol having 14 to 20 carbon atoms is preferably hexyldecanol, oleyl alcohol or octyldodecanol.

The composition of the present invention can enhance transdermal absorbability of various drugs. When the drug is a basic drug, a pharmaceutical product composition having superior transdermal absorbability can be produced by combining with the composition of the present invention. The organic acid shows an action to enhance the solubility of the basic drug in a preparation or in the skin and the transdermal permeation enhancing effect can be further improved. That is, a pharmaceutical composition superior in transdermal absorbability can be produced by combining a basic compound to be used as an active ingredient of various pharmaceutical products or a salt thereof, and the composition of the present invention. The basic drug refers to a drug having a basic functional group such as amino group (primary, secondary or tertiary) and the like in a molecule, and showing basicity as a compound. In addition, the drug is not particularly limited as long as it has the property permitting administration via the skin of a mammal such as human and the like, i.e., transdermal absorbability.

Specific examples of the drug include general anesthetics, hypnotic sedatives, antiepileptic drugs, antipyretic analgesic antiphlogistic drugs, anti-vertiginous drugs, psychoneurotic drugs, topical anesthetics, skeletal muscle relaxants, autonomic drugs, antispamodic drugs, anti-parkinsonian drugs, anti-histamine drugs, cardiac stimulants, drugs for arrhythmia, diuretics, hypotensive drugs, vasoconstrictor, coronary vasodilators, peripheral vasodilators, arteriosclerosis drugs, drugs for circulatory organ, anapnoics, antitussive expectorants, hormone drugs, external drugs for purulent diseases, analgesic-antipruritic-styptic antiphlogistic drugs, drugs for parasitic skin diseases, hemostatic drugs, drugs for treatment of gout, drugs for diabetes, antineoplastic drugs, antibiotics, chemical therapy drugs, narcotics, anti-schizophrenia drugs, antidepressants, quit smoking aids and the like.

The content of each component in the composition of the present invention can be appropriately determined according to the conditions such as the kind of drug, desired transdermal absorption rate and the like. The total weight 100 parts by weight of polyvalent alcohol, higher alcohol and organic acid contains 80-98 parts by weight, most preferably 90-97 parts by weight, of polyvalent alcohol, and the rest of higher alcohol and organic acid. The mixing ratio of the higher alcohol and organic acid (higher alcohol:organic acid) in weight ratio is 0.01:99.99-99.99:0.01, more preferably 0.1:99.9-99.9:0.1, particularly preferably 1:99-99:1, most preferably 30:70-70:30.

The composition of the present invention is used for the preparation of a transdermal absorption preparation together with a drug. Examples of the dosage form of the transdermal absorption preparation include ointment, cream, liquid, lotion, liniment, poultice, plaster, adhesive preparation and the like. In many cases, the composition of the present invention is prepared into a drug-a composition containing further contained a drug. From the aspect of transdermal drug absorption-enhancing effect, the content of the drug in the drug-a composition containing is preferably a saturation concentration or not less than 80 wt% of the saturation concentration. While a specific amount varies depending on the kind of the drug, the total weight 100 parts by weight of polyvalent alcohol, higher alcohol and organic acid preferably contains 0.1-40 parts by weight, preferably 0.5-35 parts by weight, more preferably 1.0-30 parts by weight, of a drug.

A patch preparation using the composition of the present invention is explained below.

The patch preparation of the present invention may be, what is called, a matrix type patch preparation having a drug-containing adhesive layer provided on one surface of a support or, what is called, a reservoir type patch preparation having an adhesive layer and a drug reservoir layer provided on one surface of a support.

### <Matrix type patch preparation>

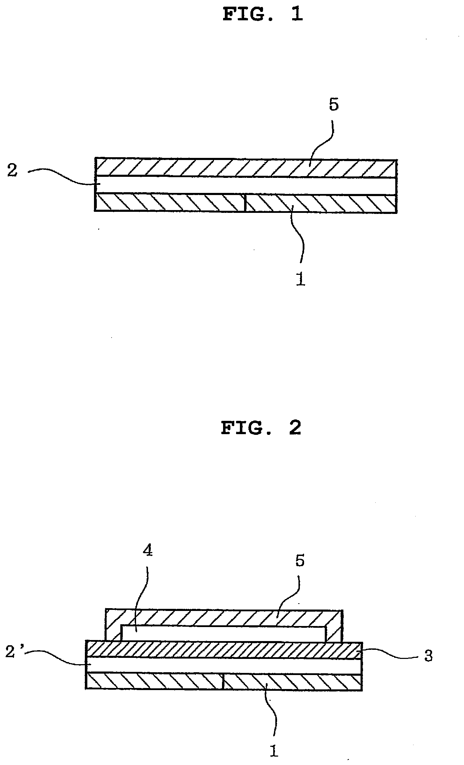

Fig. 1 shows a typical embodiment of a matrix type patch preparation of the present invention, wherein a drug-containing adhesive layer (2) and a release liner (1) are laminated in this order on one surface of a support (5). In a matrix type patch preparation, a drug-containing adhesive layer containing the composition of the present invention is formed on one surface of a support.

The drug-containing adhesive layer can be formed through the following process:
mixing the above-mentioned drug-containing composition prepared by adding a drug to the composition of the present invention, about 40 - 1900 parts by weight (preferably about 67 - 900 parts by weight) of an adhesive polymer based on the above-mentioned drug-containing composition (100 parts by weight), an adequate amount of a solvent as necessary, the below-mentioned plasticizer and the like as necessary to prepare a composition for forming an adhesive layer;
applying the composition for forming an adhesive layer onto one surface of a support or a peel-treated surface of a release liner to form a laminate; and
drying the laminate. While the solvent is not particularly limited, ethyl acetate, toluene, hexane and the like are preferable. The drug-containing adhesive layer can be crosslinked and, in this case, a crosslinking agent can be further added to the composition for forming an adhesive layer. The composition for forming an adhesive layer can be applied to one surface of a support or release liner by, for example, casting, printing, and other technique known per se to those of ordinary skill in the art. After forming a drug-containing adhesive layer, a release liner or support is preferably adhered thereto for protection, preservation and the like of the drug-containing adhesive layer.

The above-mentioned adhesive polymer is not particularly limited, and acrylic polymer containing (meth)acrylic acid ester polymer; rubber polymer such as styrene-isoprene-styrene block copolymer, styrene-butadiene-styrene block copolymer, polyisoprene, polyisobutylene, polybutadiene and the like; silicone polymer such as silicone rubber, dimethylsiloxane base, diphenylsiloxane base and the like; vinyl ether polymer such as polyvinyl methyl ether, polyvinyl ethyl ether, polyvinyl isobutyl ether and the like; vinyl ester polymer such as vinyl acetate-ethylene copolymer and the like; ester polymer consisting of carboxylic acid component such as dimethyl terephthalate, dimethyl isophthalate, dimethyl phthalate and the like, and polyvalent alcohol component such as ethylene glycol and the like and the like can be mentioned. Of these, an acrylic polymer is preferable from the aspect of compatibility with polyvalent alcohol.

As an acrylic polymer, preferred is one obtained by copolymerization of (meth)acrylic acid alkyl ester as a main component and a functional monomer. That is, a copolymer comprising 50 - 99 wt% (preferably 60 - 95 wt%) of a monomer component consisting of (meth)acrylic acid alkyl ester, wherein the rest of the monomer component is a functional monomer, is preferable. The main component here means a monomer component contained in a proportion of not less than 50 wt% of the total weight of the monomer component constituting the copolymer.

The (meth)acrylic acid alkyl ester (hereinafter to be also referred to as the main component monomer) is generally that wherein the alkyl group is a straight chain or branched chain alkyl group having 4 - 13 carbon atoms (e.g., butyl, pentyl, hexyl, heptyl, octyl, 2-ethylhexyl, nonyl, decyl, undecyl, dodecyl, tridecyl and the like), and one or more kinds thereof are used.

The functional monomer has at least one unsaturated double bond, which is involved in a copolymerization reaction, in a molecule and a functional group on the side chain. Examples thereof include carboxyl group-containing monomer such as (meth)acrylic acid, itaconic acid, maleic acid, maleic anhydride and the like, hydroxyl group-containing monomer such as (meth)acrylic acid hydroxyethyl ester, (meth)acrylic acid hydroxypropyl ester and the like; sulfoxyl group-containing monomer such as styrene sulfonic acid, allyl sulfonic acid, sulfopropyl(meth)acrylate, (meth)acryloyloxynaphthalene sulfonic acid, acrylamide methylpropane sulfonic acid and the like; amino group-containing monomer such as (meth)acrylic acid aminoethyl ester, (meth)acrylic acid dimethylaminoethyl ester, (meth)acrylic acid tert-butylaminoethyl ester and the like; amide group-containing monomer such as (meth)acrylamide, dimethyl(meth)acrylamide, N-methylol(meth)acrylamide, N-methylolpropane(meth)acrylamide, N-vinylacetamide and the like; alkoxyl group-containing monomer such as (meth)acrylic acid methoxyethyl ester, (meth)acrylic acid ethoxyethyl ester, (meth)acrylic acid methoxyethylene glycol ester, (meth)acrylic acid methoxydiethylene glycol ester, (meth)acrylic acid methoxypolyethylene glycol ester, (meth)acrylic acid methoxypolypropylene glycol ester, (meth)acrylic acid tetrahydrofuryl ester and the like.

One or more kinds of such functional monomers can be used. Of those, a carboxyl group-containing monomer is preferable, and (meth)acrylic acid is particularly preferable from the aspects of pressure-sensitive adhesiveness of an adhesive layer, cohesiveness, releaseability of a drug contained in the adhesive layer and the like.

As the acrylic polymer, one obtained by further copolymerizing the above-mentioned copolymer of (meth)acrylic acid alkyl ester (main component monomer) and a functional monomer with other monomer can also be used.

Examples of such other monomer include (meth)acrylonitrile, vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, vinyloxazole and the like. One or more kinds of these can be used.

The amount of such other monomer to be used is generally preferably about 0 - 40 wt%, more preferably about 10 - 30 wt%, relative to the total weight of the (meth)acrylic acid alkyl ester (main component monomer) and the functional monomer.

As the acrylic polymer, a terpolymer of 2-ethylhexyl acrylate as (meth)acrylic acid alkyl ester, acrylic acid and N-vinyl-2-pyrrolidone is preferable, and a copolymer obtained by copolymerizing 2-ethylhexyl acrylate, acrylic acid and N-vinyl-2-pyrrolidone at a weight ratio of 40 - 99.8:0.1 - 10:0.1 - 50, preferably 50 - 89:1 - 8:10 - 40, is more preferable, since good adhesiveness to the human skin can be achieved, and adhesion and detachment can be easily repeated.

As the rubber polymer, one containing at least one kind selected from polyisobutylene, polyisoprene and styrene-diene-styrene block copolymer (styrene-butadiene-styrene block copolymer (SBS), styrene-isoprene-styrene block copolymer (SIS) etc.) as the main component is preferable. Since high drug stability, and necessary adhesive force and cohesive force can be simultaneously achieved, a mixture of high molecular weight-polyisobutylene having a viscosity average molecular weight of 500,000 - 2,100,000, and low molecular weight-polyisobutylene having a viscosity average molecular weight of 10,000 - 200,000 at a weight ratio of 95:5 - 5:95 is particularly preferable.

When a rubber polymer is used, it is preferable to further add a tackifier, since it can improve adhesiveness of a drug-containing adhesive layer at ambient temperature. The tackifier is not particularly limited, and those known in the technical field may be appropriately selected and used. Examples thereof include petroleum resin (e.g., aromatic petroleum resin, aliphatic petroleum resin and the like), terpene resin, rosin resin, coumarone indene resin, styrene resin (e.g., styrene resin, poly(α-methylstyrene) and the like), hydrogenated petroleum resin (e.g., alicyclic saturated hydrocarbon resin and the like) and the like. Of these, an alicyclic saturated hydrocarbon resin is preferable, since the stability of the drug becomes fine. One or more kinds of tackifiers can be used in combination, and the amount of the tackifier is generally 33 - 300 wt%, preferably 50 - 200 wt%, relative to the total weight of the rubber polymer.

In the patch preparation of the present invention, the content of the composition for enhancing transdermal absorption of a drug of the present invention in the drug-containing adhesive layer is preferably 5 - 70 wt%, more preferably 10 - 60 wt%, of the drug-containing adhesive layer as 100 wt%.

When desired, the drug-containing adhesive layer can further contain a plasticizer. The plasticizer is not particularly limited as long as it plasticizes the adhesive to confer a soft feeling to the adhesive layer, and reduce the pain and skin irritation caused by the skin adhesive force during detachment of the patch preparation from the skin. When a plasticizer is added to a drug-containing adhesive layer, it is added, together with the composition of the present invention, to a composition for forming an adhesive layer during preparing of the composition. A plasticizer is preferably added in a proportion of 1 - 70 wt%, more preferably 20 - 60 wt%, of the drug-containing adhesive layer as 100 wt%.

Preferable examples of the plasticizer include fats and oils such as olive oil, castor oil, squalene, lanolin, organic solvents such as decylmethyl sulfoxide, methyloctyl sulfoxide, dimethyl sulfoxide, dimethylformamide, dimethylacetamide, methylpyrrolidone, dodecylpyrrolidone, surfactants such as polyoxyethylene sorbitan ester of fatty acid, sorbitan ester of fatty acid, polyoxyethylene fatty acid ester, phthalic acid esters such as dibutyl phthalate, diheptyl phthalate, dioctyl phthalate and the like, sebacic acid esters such as diethyl sebacate, dibutyl sebacate, dioctyl sebacate and the like, hydrocarbons such as liquid paraffin, fatty acid esters such as ethyl oleate, diisopropyl adipate, isopropyl palmitate, octyl palmitate, isopropyl myristate, isotridecyl myristate, ethyl laurate and the like, fatty acid ester of glycerin, propylene glycol ester of fatty acid , ethoxylated stearyl alcohol, pyrrolidone carboxylic acid fatty acid ester and the like. Any one kind of these may be used alone, or two or more kinds thereof may be used in combination.

A crosslinking structure can be introduced into the drug-containing adhesive layer. For this end, the drug-containing adhesive layer can be subjected to a physical crosslinking treatment by irradiation such as UV irradiation, electron beam irradiation and the like, or a chemical crosslinking treatment using various crosslinking agents such as isocyanate compounds (e.g., trifunctional isocyanates and the like), organic peroxide, organometallic salt, metal alcoholate, metal chelate compound, polyfunctional compound (polyfunctional external crosslinking agents and polyfunctional monomers for internal crosslinking such as diacrylate, dimethacrylate and the like) and the like. When a chemical crosslinking treatment is performed, a crosslinking agent is added, together with the composition of the present invention, to a composition for forming an adhesive layer, the composition for forming an adhesive layer is applied to one surface of a support or a peel-treated surface of a release liner and dried to form a drug-containing adhesive layer, the release liner or support is adhered onto the drug-containing adhesive layer, and the laminate is left standing at 60 - 90°C, preferably 60 - 70°C, for 24 - 48 hr to enhance the crosslinking reaction, whereby a drug-containing adhesive layer having a crosslinking structure is formed.

In the patch preparation of the present invention, while the thickness of the drug-containing adhesive layer is not particularly limited, it is preferably 20 - 300 µm, more preferably 30 - 300 µm, most preferably 50 - 300 µm. When the thickness of the adhesive layer is less than 20 µm, it may be difficult to obtain sufficient adhesive force and contain an effective amount of a drug. When the thickness of the adhesive layer exceeds 300 µm, formation of an adhesive layer may become difficult (difficulty of coating).

While the support is not particularly limited, it is specifically, for example, a single film such as polyester (e.g., poly(ethylene terephthalate) (PET) etc.), nylon, polyvinyl chloride, polyethylene, polypropylene, ethylene-vinyl acetate copolymer, polytetrafluoroethylene, ionomer resin and the like, metal foil, or a laminate film of two or more kinds of films selected therefrom and the like. To improve adhesiveness (anchor property) of a support to an adhesive layer, the support is preferably a laminate film of a non-porous film comprised of the above-mentioned material and a porous film mentioned below, and an adhesive layer is preferably formed on the side of the porous film. The thickness of the non-porous film is preferably 2 - 100 µm, more preferably 2 - 50 µm.

The porous film is not particularly limited as long as it improves the anchor property to an adhesive layer and, for example, paper, woven fabric, non-woven fabric (e.g., polyester (e.g., poly(ethylene terephthalate) (PET) and the like) non-woven fabric and the like), the above-mentioned film with mechanical perforation (e.g., single films such as polyester, nylon, Saran (trade name), polyethylene, polypropylene, ethylene-vinyl acetate copolymer, polyvinyl chloride, ethylene-ethyl acrylate copolymer, polytetrafluoroethylene, metal foil, poly(ethylene terephthalate) and the like, and a laminate film by laminating one or more kinds of these and the like) and the like can be mentioned. Particularly, paper, woven fabric and non-woven fabric (e.g., polyester non-woven fabric, poly(ethylene terephthalate) non-woven fabric and the like) are preferable to afford flexibility of the support. When a porous film, for example, woven fabric or non-woven fabric is used, the weight thereof is preferably 5 - 30 g/m² to improve the anchor property.

The laminate film as a support is produced by a known production method of a laminate film such as dry lamination method, wet lamination method, extrusion lamination method, hot melt lamination method, coextrusion lamination method and the like.

While the thickness of the support is not particularly limited, it is preferably 2 - 200 µm, more preferably 10 - 50 µm. When it is less than 2 µm, handling property such as self-supporting property and the like tends to decrease, and when it exceeds 200 µm, an unpleasant feeling (a feeling of stiffness) is produced to often degrade the followability.

Examples of the release liner include a release liner having a peel-treated layer comprised of a peel-treating agent, which is formed on the surface of a substrate for a release liner, a plastic film having high detachability in itself, a release liner having a release layer comprised of the aforementioned plastic film having high detachability, which is formed on the surface of a substrate for release liner and the like. The release surface of the release liner may be only one surface of the substrate or both surfaces thereof.

In such release liner, the peel-treating agent is not particularly limited and examples thereof include release agents such as long-chain alkyl group-containing polymer, silicone polymer (silicone release agent), fluorine polymer (fluorine release agent) and the like. Examples of the substrate for a release liner include plastic films such as poly(ethylene terephthalate) (PET) film, polyimide film, polypropylene film, polyethylene film, polycarbonate film, polyester (excluding PET) film and the like and metal vapor-deposited plastic film obtained by vapor deposition of a metal on these films; papers such as Japanese paper, foreign paper, craft paper, glassine, fine paper and the like; substrates made from a fibrous material such as non-woven fabric, fabric and the like; metal foil and the like.

Examples of the plastic film having high detachability in itself include ethylene-α-olefin copolymers (block copolymer or random copolymer) such as polyethylene (low density polyethylene, linear low density polyethylene etc.), polypropylene, ethylene-propylene copolymer and the like, polyolefin film made of a polyolefin resin comprised of a mixture thereof; Teflon (registered trade mark) film and the like.

A release layer on the surface of the aforementioned substrate for a release liner can be formed by laminating or coating a material of the aforementioned plastic film having high detachability on the aforementioned substrate for a release liner.

While thickness (total thickness) of the release liner is not particularly limited, it is generally not more than 200 µm, preferably 25 - 100 µm.

### <Reservoir type patch preparation>

Fig. 2 shows a typical example of the reservoir type patch preparation of the present invention, wherein a drug reservoir layer (4), a drug permeation control film (3), an adhesive layer (2'), and a release liner (1) are laminated in this order on one surface of a support (5).

In a reservoir type patch preparation the composition of the present invention is generally used for a drug reservoir layer. That is, a drug is added to the composition of the present invention to give a drug-a composition containing, which is applied to a drug reservoir layer. The drug-containing composition can further contain a drug stabilizer, a gelling agent and the like. In addition, it can also be applied to a drug reservoir layer by impregnating a non-woven fabric and the like with a drug-a composition containing.

The materials, thickness etc. to be used for support (5) and release liner (1) are basically the same as those of the aforementioned matrix type patch preparation. As a drug permeation control film (3), a micro pore film having an average pore size of 0.1 - 1 µm can be mentioned. As a material for the micro pore film, polyolefin such as polypropylene, polyethylene and the like, polytetrafluoroethylene and the like are used. While the thickness of the drug permeation control film is generally 1 µm - 200 µm, it is desirably 10 µm - 100 µm particularly from the aspects of easiness of production, appropriate stiffness and the like.

The drug-containing adhesive layer (2) in the matrix type patch preparation and the adhesive layer (2') in the reservoir type patch preparation are preferably hydrophobic adhesive layers, more preferably nonaqueous adhesive layers, from the aspect of skin adhesiveness. The nonaqueous adhesive layer here is not necessarily limited to one completely free of water, and may contain a small quantity of water derived from air humidity, skin and the like. The small quantity of water here is preferably not more than 5 wt%, more preferably not more than 2 wt%, most preferably not more than 1 wt%, as a water content of a laminate of a support and an adhesive layer. The water content of a laminate of a support and an adhesive layer here means the weight ratio of water contained in the laminate of a support and an adhesive layer (weight percentage of water relative to the total weight of the laminate of a support and an adhesive layer) as measured by Karl Fischer coulometric titration method, which is specifically as follows. That is, under an environment controlled to temperature 23±2°C and relative humidity 40±5%RH, a sample (preparation) is cut out in a given size. Since preparations are generally protected by a release liner, when the test piece has a release liner, the release liner is removed and the test piece is placed in a moisture vaporizing device. The test piece is heated at 140°C in the moisture vaporizing device, water developed thereby is introduced into a titration flask while using nitrogen as a carrier, and the water content (wt%) of the sample is measured by Karl Fischer coulometric titration method.

The present invention is explained in more detail in the following by referring to Examples and Comparative Examples, which are not to be construed as limitative. In the following description, unless otherwise specified, "parts" or "%" means "parts by weight" or "wt%".

### Examples 1-27 and Comparative Examples 1-15

### <Preparation of compositions for enhancing transdermal absorption>

The starting materials in the amounts shown in Tables 1, 2 were blended, a drug for patch preparation was added at a saturated concentration or above, and the mixture was stirred. The mixture was filtered through a polytetrafluoroethylene (PTFE) disposable filter with a pore size 0.45 µm to give a composition for enhancing transdermal absorption of a drug, which contained the drug at a saturation concentration. As the drug, a basic drug Zolmitriptan was used. In the Table, the unit of the numbers is parts by weight.

The compositions of Examples 1-27 and Comparative Examples 1-15 were subjected to the following skin permeability test to evaluate the enhancing effect on the transdermal absorption of a drug. The results are shown in Tables 3, 4.

### <Skin permeability test>

The skin isolated from a hairless mouse was mounted on a cell for skin permeation experiment (effective area 3 mmϕ) such that the stratum corneum side was a donor phase and the corium side was a receptor phase. Phosphate-buffered saline (pH 7.4) was placed in the receptor phase, a drug-containing composition for enhancing transdermal absorption was placed in the donor phase, and a skin permeation experiment was conducted at 32°C for 24 hr. The receptor solution was collected 24 hr later, and the drug concentration was measured by high performance liquid chromatography (HPLC).

### <HPLC measurement conditions> (ZLM)

column: Inertsil ODS-3 (particle size 3 µm, inner diameter 3.0 mm×length 75 mm) manufactured by GL Sciences Inc.
mobile phase: (1 g/L phosphoric acid/aqueous 50 mM ammonium acetate solution)/acetonitrile = 85/15
detection wavelength: 283 nm
flow rate: 0.45 mL/min
column temperature:40°C
analysis time:5 min
retention time: 3.0 min

**Table 1**

| | drug | polyvalent alcohol | higher alcohol | organic acid | | | | | | | |
|---|---|---|---|---|---|---|---|---|---|---|---|
| | | PG | OA | acetic acid | lactic acid | tartaric acid | citric acid | benzoic acid | myristic acid | isostearic acid | oleic acid |
| | ZLM | C3 | C18 | C2 | C3 | C4 | C6 | C7 | C14 | C18 | C18 |
| Comp. Ex. 1 | saturated | **100** | | | | | | | | | |
| Comp. Ex. 2 | saturated | | **100** | | | | | | | | |
| Comp. Ex. 3 | saturated | **98** | **2** | | | | | | | | |
| Comp. Ex. 4 | saturated | **98** | | **2** | | | | | | | |
| Comp. Ex. 5 | saturated | **98** | | | **2** | | | | | | |
| Comp. Ex. 6 | saturated | **98** | | | | **2** | | | | | |
| Comp. Ex. 7 | saturated | **98** | | | | | **2** | | | | |
| Comp. Ex. 8 | saturated | **98** | | | | | | **2** | | | |
| Comp. Ex. 9 | saturated | **98** | | | | | | | **2** | | |
| Comp. Ex. 10 | saturated | **98** | | | | | | | | **2** | |
| Comp. Ex. 11 | saturated | **98** | | | | | | | | | **2** |
| Ex. 1* | saturated | **98** | **1** | **1** | | | | | | | |
| Ex. 2* | saturated | **98** | **1** | | **1** | | | | | | |
| Ex. 3* | saturated | **98** | **1** | | | **1** | | | | | |
| Ex. 4* | saturated | **98** | **1** | | | | **1** | | | | |
| Ex. 5* | saturated | **98** | **1** | | | | | **1** | | | |
| Ex. 6 | saturated | **98** | **1** | | | | | | **1** | | |
| Ex. 7 | saturated | **98** | **1** | | | | | | | **1** | |
| Ex. 8 | saturated | **98** | **1** | | | | | | | | **1** |

| | | | | | | | | | | | |
|---|---|---|---|---|---|---|---|---|---|---|---|
| *Reference Example | | | | | | | | | | | |

The official names of the abbreviations in Table 1 are as described below.
ZLM: Zolmitriptan, PG: propylene glycol, OA: oleyl alcohol

**Table 2**

| | basic drug | polyvalent alcohol | higher alcohol | | | | organic acid | | | | | | |
|---|---|---|---|---|---|---|---|---|---|---|---|---|---|
| | ZLM | PG | LA | MA | HDE | ODO | lactic acid | tartaric acid | citric acid | benzoic acid | myristic acid | isostearic acid | oleic acid |
| | | C3 | C12 | C14 | C16 | C20 | C3 | C4 | C6 | C7 | C14 | C18 | C18 |
| Comp. Ex. 12 | saturated | **98** | **2** | | | | | | | | | | |
| Comp. Ex. 13 | saturated | **98** | | **2** | | | | | | | | | |
| Comp. Ex. 14 | saturated | **98** | | | **2** | | | | | | | | |
| Comp. Ex. 15 | saturated | **98** | | | | **2** | | | | | | | |
| Ex. 9* | saturated | **98** | **1** | | | | **1** | | | | | | |
| Ex. 10* | saturated | **98** | **1** | | | | | **1** | | | | | |
| Ex. 11* | saturated | **98** | **1** | | | | | | **1** | | | | |
| Ex. 12* | saturated | **98** | **1** | | | | | | | **1** | | | |
| Ex. 13 | saturated | **98** | **1** | | | | | | | | | | **1** |
| Ex. 14* | saturated | **98** | | **1** | | | **1** | | | | | | |
| Ex. 15* | saturated | **98** | | **1** | | | | **1** | | | | | |
| Ex. 16* | saturated | **98** | | **1** | | | | | **1** | | | | |
| Ex. 17 | saturated | **98** | | **1** | | | | | | | **1** | | |
| Ex. 18 | saturated | **98** | | **1** | | | | | | | | **1** | |
| Ex. 19 | saturated | **98** | | **1** | | | | | | | | | **1** |
| Ex. 20* | saturated | **98** | | | **1** | | **1** | | | | | | |
| Ex. 21* | saturated | **98** | | | **1** | | | | **1** | | | | |
| Ex. 22* | saturated | **98** | | | **1** | | | | | **1** | | | |
| Ex. 23 | saturated | **98** | | | **1** | | | | | | **1** | | |
| Ex. 24 | saturated | **98** | | | **1** | | | | | | | | **1** |
| Ex. 25* | saturated | **98** | | | | **1** | | | **1** | | | | |
| Ex. 26 | saturated | **98** | | | | **1** | | | | | **1** | | |
| Ex. 27 | saturated | **98** | | | | **1** | | | | | | | **1** |

| | | | | | | | | | | | | | |
|---|---|---|---|---|---|---|---|---|---|---|---|---|---|
| *Reference Example | | | | | | | | | | | | | |

The official names of the abbreviations in Table 2 are as described below.
ZLM: Zolmitriptan, PG: propylene glycol, LA: lauryl alcohol, MA: myristyl alcohol, HDE: hexyldecanol, ODO: octyldodecanol

**Table 3**

| | accumulated permeation amount (µg/cm²/24h) |
|---|---|
| Comp. Ex. 1 | **22.4** |
| Comp. Ex. 2 | **60.7** |
| Comp. Ex. 3 | **586.7** |
| Comp. Ex. 4 | **47.9** |
| Comp. Ex. 5 | **46.1** |
| Comp. Ex. 6 | **90.6** |
| Comp. Ex. 7 | **121.1** |
| Comp. Ex. 8 | **44.0** |
| Comp. Ex. 9 | **15.8** |
| Comp. Ex. 10 | **113.5** |
| Comp. Ex. 11 | **108.9** |
| Ex. 1* | **1103.7** |
| Ex. 2* | **1207.8** |
| Ex. 3* | **1619.2** |
| Ex. 4* | **1308.4** |
| Ex. 5* | **2657.5** |
| Ex. 6 | **2515.9** |
| Ex. 7 | **5520.5** |
| Ex. 8 | **4398.5** |

| | |
|---|---|
| * Reference Example | |

**[Table 4]**

| | accumulated permeation amount (µg/cm²/24h) |
|---|---|
| Comp. Ex. 12 | **2394.0** |
| Comp. Ex. 13 | **169.7** |
| Comp. Ex. 14 | **232.4** |
| Comp. Ex. 15 | **463.5** |
| Ex. 9* | **5788.1** |
| Ex. 10* | **3842.5** |
| Ex. 11* | **6158.7** |
| Ex. 12* | **4100.3** |
| Ex. 13 | **2069.3** |
| Ex. 14* | **446.3** |
| Ex. 15* | **605.0** |
| Ex. 16* | **293.3** |
| Ex. 17 | **283.9** |
| Ex. 18 | **1449.3** |
| Ex. 19 | **753.9** |
| Ex. 20* | **414.9** |
| Ex. 21* | **374.9** |
| Ex. 22* | **692.7** |
| Ex. 23 | **2398.6** |
| Ex. 24 | **3535.5** |
| Ex. 25* | **315.6** |
| Ex. 26 | **534.6** |
| Ex. 27 | **1383.9** |

| | |
|---|---|
| * Reference Example | |

In Table 3, by comparison of Reference Example 1 and Comparative Examples 1-4, the drug permeation amount was extremely low in Comparative Example 1 using propylene glycol (polyvalent alcohol) alone, Comparative Example 2 using oleyl alcohol (higher alcohol) alone, and Comparative Example 4 using propylene glycol and acetic acid (organic acid) in combination, and the drug permeability was enhanced in Comparative Example 3 using propylene glycol and oleyl alcohol in combination, as compared to Comparative Examples 1, 2. Furthermore, in Reference Example 1 using all of propylene glycol, oleyl alcohol and acetic acid in combination, the drug permeability was markedly enhanced, where the transdermal drug absorption-enhancing effect was synergistic due to the combination.

In Table 3, by comparison of Reference Example 2 and Comparative Examples 1-3, 5, the drug permeation amount was extremely low in Comparative Example 1 using propylene glycol (polyvalent alcohol) alone, Comparative Example 2 using oleyl alcohol (higher alcohol) alone, and Comparative Example 5 using propylene glycol and lactic acid (organic acid) in combination, and the drug permeability was enhanced in Comparative Example 3 using propylene glycol and oleyl alcohol in combination, as compared to Comparative Examples 1, 2. Furthermore, in Reference Example 2 using all of propylene glycol, oleyl alcohol and lactic acid in combination, the drug permeability was markedly enhanced, where the transdermal drug absorption-enhancing effect was synergistic due to the combination.

In Table 3, by comparison of Reference Example 3 and Comparative Examples 1-3, 6, the drug permeation amount was extremely low in Comparative Example 1 using propylene glycol (polyvalent alcohol) alone, Comparative Example 2 using oleyl alcohol (higher alcohol) alone, and Comparative Example 6 using propylene glycol and tartaric acid (organic acid) in combination, and the drug permeability was enhanced in Comparative Example 3 using propylene glycol and oleyl alcohol in combination, as compared to Comparative Examples 1, 2. Furthermore, in Reference Example 3 using all of propylene glycol, oleyl alcohol and tartaric acid in combination, the drug permeability was markedly enhanced, where the transdermal drug absorption-enhancing effect was synergistic due to the combination.

In Table 3, by comparison of Reference Example 4 and Comparative Examples 1-3, 7, the drug permeation amount was extremely low in Comparative Example 1 using propylene glycol (polyvalent alcohol) alone, and Comparative Example 2 using oleyl alcohol (higher alcohol) alone, and the drug permeability was enhanced in Comparative Example 7 using propylene glycol and citric acid (organic acid) in combination, and Comparative Example 3 using propylene glycol and oleyl alcohol in combination, as compared to Comparative Examples 1, 2. Furthermore, in Reference Example 4 using all of propylene glycol, oleyl alcohol and citric acid in combination, the drug permeability was markedly enhanced, where the transdermal drug absorption-enhancing effect was synergistic due to the combination.

In Table 3, by comparison of Reference Example 5 and Comparative Examples 1-3, 8, the drug permeation amount was extremely low in Comparative Example 1 using propylene glycol (polyvalent alcohol) alone, Comparative Example 2 using oleyl alcohol (higher alcohol) alone, and Comparative Example 8 using propylene glycol and benzoic acid (organic acid) in combination, and the drug permeability was enhanced in Comparative Example 3 using propylene glycol and oleyl alcohol in combination, as compared to Comparative Examples 1, 2. Furthermore, in Reference Example 5 using all of propylene glycol, oleyl alcohol and benzoic acid in combination, the drug permeability was markedly enhanced, where the transdermal drug absorption-enhancing effect was synergistic due to the combination.

In Table 3, by comparison of Example 6 and Comparative Examples 1-3, 9, the drug permeation amount was extremely low in Comparative Example 1 using propylene glycol (polyvalent alcohol) alone, Comparative Example 2 using oleyl alcohol (higher alcohol) alone, and Comparative Example 9 using propylene glycol and myristic acid (organic acid) in combination, and the drug permeability was enhanced in Comparative Example 3 using propylene glycol and oleyl alcohol in combination, as compared to Comparative Examples 1, 2. Furthermore, in Example 6 using all of propylene glycol, oleyl alcohol and myristic acid in combination, the drug permeability was markedly enhanced, where the transdermal drug absorption-enhancing effect was synergistic due to the combination.

In Table 3, by comparison of Example 7 and Comparative Examples 1-3, 10, the drug permeation amount was extremely low in Comparative Example 1 using propylene glycol (polyvalent alcohol) alone, and Comparative Example 2 using oleyl alcohol (higher alcohol) alone, and the drug permeability was enhanced in Comparative Example 10 using propylene glycol and isostearic acid (organic acid) in combination, and Comparative Example 3 using propylene glycol and oleyl alcohol in combination, as compared to Comparative Examples 1, 2. Furthermore, in Example 7 using all of propylene glycol, oleyl alcohol and isostearic acid in combination, the drug permeability was markedly enhanced, where the transdermal drug absorption-enhancing effect was synergistic due to the combination.

In Table 3, by comparison of Example 8 and Comparative Examples 1-3, 11, the drug permeation amount was extremely low in Comparative Example 1 using propylene glycol (polyvalent alcohol) alone, and Comparative Example 2 using oleyl alcohol (higher alcohol) alone, and the drug permeability was enhanced in Comparative Example 11 using propylene glycol and oleic acid (organic acid) in combination, and Comparative Example 3 using propylene glycol and oleyl alcohol in combination, as compared to Comparative Examples 1, 2. Furthermore, in Example 8 using all of propylene glycol, oleyl alcohol and oleic acid in combination, the drug permeability was markedly enhanced, where the transdermal drug absorption-enhancing effect was synergistic due to the combination.

In Tables 3, 4, by comparison of Reference Example 9 and Comparative Examples 1, 5, 12, the drug permeation amount was extremely low in Comparative Example 1 using propylene glycol (polyvalent alcohol) alone, and Comparative Example 5 using propylene glycol and lactic acid (organic acid) in combination, and the drug permeability was enhanced in Comparative Example 12 using propylene glycol and lauryl alcohol (higher alcohol) in combination, as compared to Comparative Examples 1, 5. Furthermore, in Reference Example 9 using all of propylene glycol, lauryl alcohol and lactic acid in combination, the drug permeability was markedly enhanced, where the transdermal drug absorption-enhancing effect was synergistic due to the combination.

In Tables 3, 4, by comparison of Reference Example 10 and Comparative Examples 1, 6, 12, the drug permeation amount was extremely low in Comparative Example 1 using propylene glycol (polyvalent alcohol) alone, and Comparative Example 6 using propylene glycol and tartaric acid (organic acid) in combination, and the drug permeability was enhanced in Comparative Example 12 using propylene glycol and lauryl alcohol (higher alcohol) in combination, as compared to Comparative Examples 1, 6. Furthermore, in Reference Example 10 using all of propylene glycol, lauryl alcohol and tartaric acid in combination, the drug permeability was markedly enhanced, where the transdermal drug absorption-enhancing effect was synergistic due to the combination.

In Tables 3, 4, by comparison of Reference Example 11 and Comparative Examples 1, 7, 12, the drug permeation amount was extremely low in Comparative Example 1 using propylene glycol (polyvalent alcohol) alone, but the drug permeability was enhanced in Comparative Example 7 using propylene glycol and citric acid (organic acid) in combination. In addition, the drug permeability was also enhanced in Comparative Example 12 using propylene glycol and lauryl alcohol (higher alcohol) in combination, as compared to Comparative Example 1. Furthermore, in Reference Example 11 using all of propylene glycol, lauryl alcohol and citric acid in combination, the drug permeability was markedly enhanced, where the transdermal drug absorption-enhancing effect was synergistic due to the combination.

In Tables 3, 4, by comparison of Reference Example 12 and Comparative Examples 1, 8, 12, the drug permeation amount was extremely low in Comparative Example 1 using propylene glycol (polyvalent alcohol) alone, and Comparative Example 8 using propylene glycol and benzoic acid (organic acid) in combination, and the drug permeability was enhanced in Comparative Example 12 using propylene glycol and lauryl alcohol (higher alcohol) in combination, as compared to Comparative Examples 1, 8. Furthermore, in Reference Example 12 using all of propylene glycol, lauryl alcohol and benzoic acid in combination, the drug permeability was markedly enhanced, where the transdermal drug absorption-enhancing effect was synergistic due to the combination.

In Tables 3, 4, by comparison of Example 13 and Comparative Examples 1, 11, 12, the drug permeation amount was extremely low in Comparative Example 1 using propylene glycol (polyvalent alcohol) alone, and Comparative Example 8 using propylene glycol and oleic acid (organic acid) in combination, and the drug permeability was enhanced in Comparative Example 12 using propylene glycol and lauryl alcohol (higher alcohol) in combination, as compared to Comparative Examples 1, 8. Furthermore, in Example 13 using all of propylene glycol, lauryl alcohol and oleic acid in combination, the drug permeability was enhanced, where the transdermal drug absorption-enhancing effect was synergistic due to the combination.

In Tables 3, 4, by comparison of Reference Example 14 and Comparative Examples 1, 5, 13, the drug permeation amount was extremely low in Comparative Example 1 using propylene glycol (polyvalent alcohol) alone, and Comparative Example 5 using propylene glycol and lactic acid (organic acid) in combination, and the drug permeability was enhanced in Comparative Example 13 using propylene glycol and myristyl alcohol (higher alcohol) in combination, as compared to Comparative Examples 1, 5. Furthermore, in Reference Example 14 using all of propylene glycol, myristyl alcohol and lactic acid in combination, the drug permeability was markedly enhanced, where the transdermal drug absorption-enhancing effect was synergistic due to the combination.

In Tables 3, 4, by comparison of Reference Example 15 and Comparative Examples 1, 6, 13, the drug permeation amount was extremely low in Comparative Example 1 using propylene glycol (polyvalent alcohol) alone, and Comparative Example 6 using propylene glycol and tartaric acid (organic acid) in combination, and the drug permeability was enhanced in Comparative Example 13 using propylene glycol and myristyl alcohol (higher alcohol) in combination, as compared to Comparative Examples 1, 6. Furthermore, in Reference Example 15 using all of propylene glycol, myristyl alcohol and tartaric acid in combination, the drug permeability was markedly enhanced, where the transdermal drug absorption-enhancing effect was synergistic due to the combination.

In Tables 3, 4, by comparison of Reference Example 16 and Comparative Examples 1, 7, 13, the drug permeation amount was extremely low in Comparative Example 1 using propylene glycol (polyvalent alcohol) alone, but the drug permeability was enhanced in Comparative Example 7 using propylene glycol and citric acid (organic acid) in combination, and Comparative Example 13 using propylene glycol and myristyl alcohol (higher alcohol) in combination, as compared to Comparative Example 1. Furthermore, in Reference Example 16 using all of propylene glycol, myristyl alcohol and citric acid in combination, the drug permeability was markedly enhanced, where the transdermal drug absorption-enhancing effect was synergistic due to the combination.

In Tables 3, 4, by comparison of Example 17 and Comparative Examples 1, 9, 13, the drug permeation amount was extremely low in Comparative Example 1 using propylene glycol (polyvalent alcohol) alone, and Comparative Example 9 using propylene glycol and myristic acid (organic acid) in combination, and the drug permeability was enhanced in Comparative Example 13 using propylene glycol and myristyl alcohol (higher alcohol) in combination, as compared to Comparative Examples 1, 9. Furthermore, in Example 17 using all of propylene glycol, myristyl alcohol and myristic acid in combination, the drug permeability was markedly enhanced, where the transdermal drug absorption-enhancing effect was synergistic due to the combination.

In Tables 3, 4, by comparison of Example 18 and Comparative Examples 1, 10, 13, the drug permeation amount was extremely low in Comparative Example 1 using propylene glycol (polyvalent alcohol) alone, but the drug permeability was enhanced in Comparative Example 10 using propylene glycol and isostearic acid (organic acid) in combination, and Comparative Example 13 using propylene glycol and myristyl alcohol (higher alcohol) in combination, as compared to Comparative Example 1. Furthermore, in Example 18 using all of propylene glycol, myristyl alcohol and isostearic acid in combination, the drug permeability was markedly enhanced, where the transdermal drug absorption-enhancing effect was synergistic due to the combination.

In Tables 3, 4, by comparison of Example 19 and Comparative Examples 1, 11, 13, the drug permeation amount was extremely low in Comparative Example 1 using propylene glycol (polyvalent alcohol) alone, but the drug permeability was enhanced in Comparative Example 11 using propylene glycol and oleic acid (organic acid) in combination, and Comparative Example 13 using propylene glycol and myristyl alcohol (higher alcohol) in combination, as compared to Comparative Example 1. Furthermore, in Example 19 using all of propylene glycol, myristyl alcohol and oleic acid in combination, the drug permeability was markedly enhanced, where the transdermal drug absorption-enhancing effect was synergistic due to the combination.

In Tables 3, 4, by comparison of Reference Example 20 and Comparative Examples 1, 5, 14, the drug permeation amount was extremely low in Comparative Example 1 using propylene glycol (polyvalent alcohol) alone, and Comparative Example 5 using propylene glycol and lactic acid (organic acid) in combination, and the drug permeability was enhanced in Comparative Example 14 using propylene glycol and hexyldecanol (higher alcohol) in combination, as compared to Comparative Examples 1, 5. Furthermore, in Reference Example 20 using all of propylene glycol, hexyldecanol and lactic acid in combination, the drug permeability was markedly enhanced, where the transdermal drug absorption-enhancing effect was synergistic due to the combination.

In Tables 3, 4, by comparison of Reference Example 21 and Comparative Examples 1, 7, 14, the drug permeation amount was extremely low in Comparative Example 1 using propylene glycol (polyvalent alcohol) alone, but the drug permeability was enhanced in Comparative Example 7 using propylene glycol and citric acid (organic acid) in combination, and Comparative Example 14 using propylene glycol and hexyldecanol (higher alcohol) in combination, as compared to Comparative Example 1. Furthermore, in Reference Example 21 using all of propylene glycol, hexyldecanol and citric acid in combination, the drug permeability was markedly enhanced, where the transdermal drug absorption-enhancing effect was synergistic due to the combination.

In Tables 3, 4, by comparison of Reference Example 22 and Comparative Examples 1, 8, 14, the drug permeation amount was extremely low in Comparative Example 1 using propylene glycol (polyvalent alcohol) alone, and Comparative Example 8 using propylene glycol and benzoic acid (organic acid) in combination, and the drug permeability was enhanced in Comparative Example 14 using propylene glycol and hexyldecanol (higher alcohol) in combination, as compared to Comparative Examples 1, 8. Furthermore, in Reference Example 22 using all of propylene glycol, hexyldecanol and benzoic acid in combination, the drug permeability was markedly enhanced, where the transdermal drug absorption-enhancing effect was synergistic due to the combination.

In Tables 3, 4, by comparison of Example 23 and Comparative Examples 1, 9, 14, the drug permeation amount was extremely low in Comparative Example 1 using propylene glycol (polyvalent alcohol) alone, and Comparative Example 9 using propylene glycol and myristic acid (organic acid) in combination, and the drug permeability was enhanced in Comparative Example 14 using propylene glycol and hexyldecanol (higher alcohol) in combination, as compared to Comparative Examples 1, 9. Furthermore, in Example 23 using all of propylene glycol, hexyldecanol and myristic acid in combination, the drug permeability was markedly enhanced, where the transdermal drug absorption-enhancing effect was synergistic due to the combination.

In Tables 3, 4, by comparison of Example 24 and Comparative Examples 1, 11, 14, the drug permeation amount was extremely low in Comparative Example 1 using propylene glycol (polyvalent alcohol) alone, but the drug permeability was enhanced in Comparative Example 11 using propylene glycol and oleic acid (organic acid) in combination, and Comparative Example 14 using propylene glycol and hexyldecanol (higher alcohol) in combination, as compared to Comparative Example 1. Furthermore, in Example 24 using all of propylene glycol, hexyldecanol and oleic acid in combination, the drug permeability was markedly enhanced, where the transdermal drug absorption-enhancing effect was synergistic due to the combination.

In Tables 3, 4, by comparison of Reference Example 25 and Comparative Examples 1, 7, 15, the drug permeation amount was extremely low in Comparative Example 1 using propylene glycol (polyvalent alcohol) alone, but the drug permeability was enhanced in Comparative Example 7 using propylene glycol and citric acid (organic acid) in combination, and Comparative Example 15 using propylene glycol and octyldodecanol (higher alcohol) in combination, as compared to Comparative Example 1. Furthermore, in Reference Example 25 using all of propylene glycol, octyldodecanol and citric acid in combination, a synergistic transdermal absorption-enhancing effect due to the combination was found.

In Tables 3, 4, by comparison of Example 26 and Comparative Examples 1, 9, 15, the drug permeation amount was extremely low in Comparative Example 1 using propylene glycol (polyvalent alcohol) alone, and Comparative Example 9 using propylene glycol and myristic acid (organic acid) in combination, and the drug permeability was enhanced in Comparative Example 15 using propylene glycol and octyldodecanol (higher alcohol) in combination, as compared to Comparative Examples 1, 9. Furthermore, in Example 26 using all of propylene glycol, octyldodecanol and myristic acid in combination, the drug permeability was markedly enhanced, where the enhancing effect on transdermal absorption was synergistic due to the combination.

In Tables 3, 4, by comparison of Example 27 and Comparative Examples 1, 11, 15, the drug permeation amount was extremely low in Comparative Example 1 using propylene glycol (polyvalent alcohol) alone, but the drug permeability was enhanced in Comparative Example 11 using propylene glycol and oleic acid (organic acid) in combination, and Comparative Example 15 using propylene glycol and octyldodecanol (higher alcohol) in combination, as compared to Comparative Example 1. Furthermore, in Example 27 using all of propylene glycol, octyldodecanol and oleic acid in combination, the drug permeability was markedly enhanced, where the enhancing effect on transdermal absorption was synergistic due to the combination.

### Example 28 and Comparative Examples 16-18

### <Preparation of compositions for enhancing transdermal absorption>

The starting materials in the amounts shown in Table 5 were blended, a drug for patch preparation was added at a saturated concentration or above, and the mixture was stirred. The mixture was filtered through a polytetrafluoroethylene (PTFE) disposable filter with a pore size 0.45 µm to give a composition for enhancing transdermal absorption of a drug, which contained the drug at a saturation concentration. As the drug, a basic compound Zolmitriptan was used. In the Table, the unit of the numbers is parts by weight.

The compositions of Example 28 and Comparative Examples 16-18 were subjected to the aforementioned skin permeability test to evaluate the enhancing effect on the transdermal absorption of a drug. The results are shown in Table 6.

**[Table 5]**

| | drug | polyvalent alcohol | higher alcohol | organic acid |
|---|---|---|---|---|
| | basic | BG | OA | isostearic acid |
| | ZLM | C4 | C18 | C18 |
| Comp. Ex. 16 | saturated | **100** | | |
| Comp. Ex. 17 | saturated | **98** | **2** | |
| Comp. Ex. 18 | saturated | **98** | | **2** |
| Ex. 28 | saturated | **98** | **1** | **1** |

The official names of the abbreviations in Table 5 are as described below.
ZLM: Zolmitriptan, BG: butylene glycol, OA: oleyl alcohol

**[Table 6]**

| | accumulated permeation amount (µg/cm²/24h) |
|---|---|
| Comp. Ex. 16 | **1.2** |
| Comp. Ex. 17 | **653.8** |
| Comp. Ex. 18 | **24.8** |
| Ex. 28 | **2493.7** |

In Table 6, by comparison of Example 28 and Comparative Examples 16-18, the drug permeation amount was extremely low in Comparative Example 16 using butylene glycol (polyvalent alcohol) alone and Comparative Example 18 using butylene glycol and isostearic acid (organic acid) in combination, but the drug permeability was enhanced in Comparative Example 17 using butylene glycol and oleyl alcohol in combination, as compared to Comparative Example 16. Furthermore, in Example 28 using all of butylene glycol, oleyl alcohol and isostearic acid in combination, the drug permeability was markedly enhanced, where the enhancing effect on transdermal absorption was synergistic due to the combination. Therefrom it was clarified that a combination of polyvalent alcohol, higher alcohol, and organic acid can strikingly improve the permeability of a basic drug.

### Comparative Examples 19-22

### <Preparation of compositions for enhancing transdermal absorption>

The starting materials in the amounts shown in Table 7 were blended, a drug for patch preparation was added at a saturated concentration or above, and the mixture was stirred. The mixture was filtered through a polytetrafluoroethylene (PTFE) disposable filter with a pore size 0.45 µm to give a composition for enhancing transdermal absorption of a drug, which contained the drug at a saturation concentration. As the drug, a basic compound Zolmitriptan was used. In the Table, the unit of the numbers is parts by weight.

The compositions of Comparative Examples 19-22 were subjected to the aforementioned skin permeability test. The results are shown in Table 8.

**[Table 7]**

| | drug | fatty acid ester | higher alcohol | organic acid |
|---|---|---|---|---|
| | basic | IPM | OA | isostearic acid |
| | ZLM | C17 | C18 | C18 |
| Comp. Ex. 19 | saturated | **100** | | |
| Comp. Ex. 20 | saturated | **98** | **2** | |
| Comp. Ex. 21 | saturated | **98** | | **2** |
| Comp. Ex. 22 | saturated | **98** | **1** | **1** |

The official names of the abbreviations in Table 7 are as described below.
ZLM: Zolmitriptan, IPM: isopropyl myristate, OA: oleyl alcohol

**[Table 8]**

| | accumulated permeation amount (µg/cm²/24h) |
|---|---|
| Comp. Ex. 19 | **3.9** |
| Comp. Ex. 20 | **39.7** |
| Comp. Ex. 21 | **22.9** |
| Comp. Ex. 22 | **29.1** |

In Table 8, by comparison of the results of Comparative Example 19-22, the drug permeation amount was extremely low in Comparative Example 19 using isopropyl myristate (fatty acid ester) alone, and Comparative Example 20 using isopropyl myristate and oleyl alcohol in combination and Comparative Example 21 using isopropyl myristate and isostearic acid (organic acid) in combination showed improved drug permeation as compared to Comparative Example 19. Although drug permeability was improved in Comparative Example 22 using all of isopropyl myristate, oleyl alcohol, and isostearic acid in combination, as compared to Comparative Example 19 using isopropyl myristate alone, a synergistic enhancing effect on transdermal drug absorption could not be observed by a combination of oleyl alcohol and isostearic acid. Therefore, it was clarified from this and the aforementioned results of Examples 1-28 that a synergistic drug permeability improving effect by a combination of a higher alcohol and an organic acid cannot be achieved unless a polyvalent alcohol, a higher alcohol and an organic acid are combined.

### Example 29 and Comparative Examples 23-25

### <Preparation of compositions for enhancing transdermal absorption>

The starting materials in the amounts shown in Table 9 were blended, a drug for patch preparation was added at a saturated concentration or above, and the mixture was stirred. The mixture was filtered through a polytetrafluoroethylene (PTFE) disposable filter with a pore size 0.45 µm to give a composition for enhancing transdermal absorption of a drug, which contained the drug at a saturation concentration. As the drug, a basic compound propranolol was used. In the Table, the unit of the numbers is parts by weight.

The compositions of Example 29 and Comparative Example 23-25 were subjected to the aforementioned skin permeability test to evaluate the enhancing effect on the transdermal absorption of a drug. The concentration of the drug in the receptor solution was measured by HPLC under the following conditions. The results are shown in Table 10.

### <HPLC measurement conditions> (PRP)

column: Inertsil ODS-3 (particle size 3 µm, inner diameter 3.0 mm×length 75 mm
manufactured by GL Sciences Inc.
mobile phase: phosphate buffer (pH 2.5)/acetonitrile=70/30
detection wavelength: 292 nm
flow rate: 0.40 mL/min
column temperature: 40°C
analysis time: 5 min
retention time: 3.2 min

**[Table 9]**

| | drug | polyvalent alcohol | higher alcohol | organic acid |
|---|---|---|---|---|
| | basic | PG | OA | isostearic acid |
| | PRP | C3 | C18 | C18 |
| Comp. Ex. 23 | saturated | **100** | | |
| Comp. Ex. 24 | saturated | **98** | **2** | |
| Comp. Ex. 25 | saturated | **98** | | **2** |
| Ex. 29 | saturated | **98** | **1** | **1** |

The official names of the abbreviations in Table 9 are as described below.
PRP: propranolol, PG: propylene glycol, OA: oleyl alcohol

**[Table 10]**

| | accumulated permeation amount (µg/cm²/24h) |
|---|---|
| Comp. Ex. 23 | **1019.1** |
| Comp. Ex. 24 | **5446.1** |
| Comp. Ex. 25 | **1558.0** |
| Ex. 29 | **7929.3** |

In Table 10, by comparison of Example 29 and Comparative Examples 23-25, the drug permeation was enhanced in Comparative Example 25 using propylene glycol and isostearic acid (organic acid) in combination, and Comparative Example 24 using propylene glycol and oleyl alcohol (higher alcohol) in combination, as compared to Comparative Example 23 using propylene glycol (polyvalent alcohol) alone. Furthermore, in Example 29 using all of propylene glycol, oleyl alcohol, and isostearic acid in combination, the drug permeation was markedly enhanced, where the enhancing effect on transdermal drug absorption was synergistic. Therefrom it was clarified that a combination of a polyvalent alcohol, a higher alcohol and an organic acid can remarkably improve the skin permeability of a basic drug.

### Reference Example 30 and Comparative Examples 26-28

### <Preparation of compositions for enhancing transdermal absorption>

The starting materials in the amounts shown in Table 11 were blended, a drug for patch preparation was added at a saturated concentration or above, and the mixture was stirred. The mixture was filtered through a polytetrafluoroethylene (PTFE) disposable filter with a pore size 0.45 µm to give a composition for enhancing transdermal absorption of a drug, which contained the drug at a saturation concentration. As the drug, an acidic drug indomethacin was used. In the Table, the unit of the numbers is parts by weight.

The compositions of Reference Example 30 and Comparative Example 26-28 were subjected to the aforementioned skin permeability test. The concentration of the drug in the receptor solution was measured by HPLC under the following conditions. The results are shown in Table 12.

### <HPLC measurement conditions> (IND)

column: Inertsil ODS-3 (particle size 3 µm, inner diameter 3.0 mm×length 75 mm) manufactured by GL Sciences Inc.
mobile phase: methanol/1 g/L aqueous phosphoric acid solution=70/30
detection wavelength: 254 nm
flow rate: 0.70 mL/min
column temperature: 40°C
analysis time: 5 min
retention time: 3.4 min

**[Table 11]**

| | drug | polyvalent alcohol | higher alcohol | organic acid |
|---|---|---|---|---|
| | acidic | PG | OA | isostearic acid |
| | IND | C3 | C18 | C18 |
| Comp. Ex. 26 | saturated | **100** | | |
| Comp. Ex. 27 | saturated | **98** | **2** | |
| Comp. Ex. 28 | saturated | **98** | | **2** |
| Ex. 30* | saturated | **98** | **1** | **1** |

| | | | | |
|---|---|---|---|---|
| *Reference Example | | | | |

The official names of the abbreviations in Table 11 are as described below.
IND: indomethacin, PG: propylene glycol, OA: oleyl alcohol.

**[Table 12]**

| | accumulated permeation amount (µg/cm²/24h) |
|---|---|
| Comp. Ex. 26 | **18.3** |
| Comp. Ex. 27 | **1356.6** |
| Comp. Ex. 28 | **73.3** |
| Ex. 30* | **1331.0** |

| | |
|---|---|
| *Reference Example | |

In Table 12, by comparison of Reference Example 30 and Comparative Examples 26-28, the drug permeation amount was low in Comparative Example 26 using propylene glycol (polyvalent alcohol) alone, and Comparative Example 28 using propylene glycol and isostearic acid (organic acid) in combination. However, drug permeation was enhanced in Comparative Example 27 using propylene glycol and oleyl alcohol (higher alcohol) in combination. Moreover, the transdermal drug absorption-enhancing effect was a synergistic effect due to the combination in Reference Example 30 using all of propylene glycol, oleyl alcohol, and isostearic acid in combination. Therefrom it was clarified that the transdermal drug absorption-enhancing effect by a combination of polyvalent alcohol, higher alcohol, and organic acid is synergistic in acidic drugs as well.

### <Formulation of patch preparation>

### (1) Preparation of acrylic polymer solution

Under an inert gas atmosphere, 2-ethylhexyl acrylate (75 parts), N-vinyl-2-pyrrolidone (22 parts), acrylic acid (3 parts) and azobisisobutyronitrile (0.2 part) were added to ethyl acetate, and solution polymerization was performed at 60°C to give an acrylic polymer solution (polymer solid content: 28%).

### (2) Preparation of polyisobutylene polymer solution

Polymer polyisobutylene (viscosity average molecular weight 4,000,000, Oppanol(R) B200, manufactured by BASF, 22.0 parts as a solid content), low molecular weight polyisobutylene (viscosity average molecular weight 55,000, Oppanol(R) B12, manufactured by BASF, 38.0 parts as a solid content), and an alicyclic saturated hydrocarbon resin (softening point 140°C, ARKON(R) P-140, manufactured by Arakawa Chemical Industries, Ltd., 40.0 parts) were dissolved in toluene to give a polyisobutylene polymer solution (polymer solid content: 21%).

### (3) Preparation of patch preparation

Of 100 parts of the above-mentioned composition for patch preparation, 50 parts of propylene glycol was replaced by an acrylic polymer solution containing 49.7 parts of the solid content and 0.3 part of a crosslinking agent, or a polyisobutylene polymer solution containing 50 parts of the solid content to give a composition for an adhesive layer formation (coating solution). This is applied to one surface of a poly(ethylene terephthalate) (hereinafter to be referred to as PET) film (thickness 75 µm) as a release liner, such that the thickness after drying is 200 µm, and dried to form an adhesive layer. To the adhesive layer is adhered a non-woven fabric surface of a PET film (thickness 2 µm)-PET non-woven fabric (fabric weight 12 g/m²) laminate as a support and, when an acrylic polymer is used, the laminate is subjected to an aging treatment (crosslinking treatment of adhesive layer) at 70°C for 48 hr to give a laminated sheet. The laminated sheet is cut into a shape of a patch preparation, and packed in a package container in an atmosphere with an oxygen concentration of 3% or below to give a patch preparation. The patch preparation of the present invention shows superior drug transdermal absorbability by the superior transdermal drug absorption-enhancing effect of the above-mentioned composition for forming patch preparation.

Since the composition of the present invention can increase the transdermal absorbability of a basic drug, a transdermal absorption preparation of a basic drug, which has heretofore been difficult to formulate due to its low transdermal absorbability, can be formulated by applying the composition of the present invention.

This application is based on a patent application No. 2013-048450 filed in Japan.

## Claims

1. A composition for enhancing transdermal absorption of a basic drug, comprising a polyvalent alcohol having 3 to 8 carbon atoms, an organic acid having 12 to 20 carbon atoms, and a higher alcohol having 12 to 20 carbon atoms,
wherein the amount of the polyvalent alcohol is 80-98 parts by weight per 100 parts by weight of the total weight of the polyvalent alcohol, higher alcohol and organic acid, and the weight ratio of the higher alcohol and organic acid (higher alcohol:organic acid) in the remaining part is 0.01:99.99-99.99:0.01.

2. The composition according to claim 1, wherein the higher alcohol having 12 to 20 carbon atoms is one or more kinds selected from the group consisting of lauryl alcohol, myristyl alcohol, hexyldecanol, oleyl alcohol and octyldodecanol.

3. The composition according to claim 1, wherein the higher alcohol having 12 to 20 carbon atoms is a higher alcohol having 14 to 20 carbon atoms.

4. The composition according to any one of claims 1 to 3, which is for a patch preparation.

5. A patch preparation comprising a support and a drug-containing adhesive layer or drug reservoir layer on one surface of the support, wherein the layer comprises the composition according to any one of claims 1 to 3 and a basic drug.

6. A composition according to any one of claims 1 to 4 for use to enhance transdermal absorption of a basic drug.

## Patentansprüche

1. Zusammensetzung zur Erhöhung der transdermalen Resorption eines basischen Wirkstoffs, umfassend einen mehrwertigen Alkohol mit 3 bis 8 Kohlenstoffatomen, einer organischen Säure mit 12 bis 20 Kohlenstoffatomen und einem höheren Alkohol mit 12 bis 20 Kohlenstoffatomen;
wobei die Menge des mehrwertigen Alkohols 80-98 Gewichtsteile pro 100 Gewichtsteile des Gesamtgewichts des mehrwertigen Alkohols, des höheren Alkohols und der organischen Säure beträgt und das Gewichtsverhältnis des höheren Alkohols zu der organischen Säure (höherer Alkohol:organische Säure) im restlichen Teil 0,01:99,99 bis 99,99:0,01 beträgt.

2. Zusammensetzung gemäß Anspruch 1, wobei es sich bei dem höheren Alkohol mit 12 bis 20 Kohlenstoffatomen um eine oder mehrere Arten handelt, die aus der Gruppe ausgewählt sind, die aus Laurylalkohol, Myristylalkohol, Hexyldecanol, Oleylalkohol und Octyldodecanol besteht.

3. Zusammensetzung gemäß Anspruch 1, wobei der höhere Alkohol mit 12 bis 20 Kohlenstoffatomen ein höherer Alkohol mit 14 bis 20 Kohlenstoffatomen ist.

4. Zusammensetzung gemäß einem der Ansprüche 1 bis 3, die für ein Pflasterpräparat dient.

5. Pflasterpräparat, umfassend einen Träger und eine wirkstoffhaltige Kleberschicht oder Wirkstoffreservoirschicht auf einer Oberfläche des Trägers, wobei die Schicht die Zusammensetzung gemäß einem der Ansprüche 1 bis 3 und einen basischen Wirkstoff umfasst.

6. Zusammensetzung gemäß einem der Ansprüche 1 bis 4 zur Verwendung zur Erhöhung der transdermalen Resorption eines basischen Wirkstoffs.

## Revendications

1. Composition pour amplifier l'absorption transdermique d'un médicament basique, comprenant un alcool polyvalent comportant 3 à 8 atomes de carbone, un acide organique comportant 12 à 20 atomes de carbone, et un alcool supérieur comportant 12 à 20 atomes de carbone,
dans laquelle la quantité de l'alcool polyvalent est de 80 à 98 parties en poids pour 100 parties en poids du poids total de l'alcool polyvalent, de l'alcool supérieur et de l'acide organique, et le rapport pondéral de l'alcool supérieur et de l'acide organique (alcool supérieur:acide organique) dans la partie restante est de 0,01:99,99 à 99,99:0,01.

2. Composition selon la revendication 1, dans laquelle l'alcool supérieur comportant 12 à 20 atomes de carbone est d'un ou de plusieurs types choisis dans le groupe constitué de l'alcool laurylique, l'alcool myristylique, l'hexyldécanol, l'alcool oléylique et l'octyldodécanol.

3. Composition selon la revendication 1, dans laquelle l'alcool supérieur comportant 12 à 20 atomes de carbone est un alcool supérieur comportant 14 à 20 atomes de carbone.

4. Composition selon l'une quelconque des revendications 1 à 3, qui est pour une préparation de patch.

5. Préparation de patch comprenant un support et une couche adhésive contenant un médicament ou une couche de réservoir de médicament sur une surface du support, dans laquelle la couche comprend la composition selon l'une quelconque des revendications 1 à 3 et un médicament basique.

6. Composition selon l'une quelconque des revendications 1 à 4 pour une utilisation pour amplifier l'absorption transdermique d'un médicament basique.
